# EUROPEAN PATENT APPLICATION

(11) **EP 1 072 265 A1**
(43) Date of publication of application: **31.01.2001**
(21) Application number: 99830464.6
(22) Date of filing: 20.07.1999
(51) Int. Cl.: A61K 31/352, A61P 39/04

(54) **Use of plant polyphenols for treating iron overload**

(71) Applicant: MEDIS S.r.l. Medical Infusion Systems, 20127 Milano (IT)
(72) Inventor: Ghisalberti, Carlo, Dr., 20154 Milano (IT)
(74) Representative: Bianchetti, Giuseppe, Prof.

(57) **Abstract**

Compositions and a method of treating iron overloading in human subjects are described, said treatment being carried out by means of catechic- and flavonoid-structure plant polyphenols, orally administered alone or in combination thereof, or with common nutritional supplements to enhance the efficacy of prevention of the oxidative metabolic damages caused by excess iron.

## Description

### Technical field

The present invention relates to compositions and a method of treating iron overloading in human subjects by means of catechic- and flavonoid-structure plant polyphenols, orally administered alone or in combination thereof, or with common nutritional supplements to enhance the efficacy of prevention of the oxidative metabolic damages caused by excess iron.

### Background of the invention

Iron overloading is a relatively common condition leading to complicances due to the oxidative stress catalyzed by excess body iron, promoting the formation of free radicals, thus damaging various body sites such as biomolecules (e.g. hormones), biological fluids (e.g. synovial fluid), tissues (e.g. liver, pancreas) and cardiovascular system, mainly to the heart, causing arithmias and heart failures.

The iron overload condition is also named hemosiderosis, siderosis, hemochromatosis, or chromatosis, and it can have a variety of etiologies.

The chronic treatment based on blood transfusions is usually mandatory in subjects with refractory anemia, thalassemia major, aplastic anemia, congenital (Blackfan-Diamond) anemia, acquired red cell aplasia, and may be necessary in subjects with diseases such as thalassemia intermedia, sickle cell anemia, and myelodisplasia. The repeated transfusions cause iron overloading, which in turn is the origin of a number of clinical complicances.

Further iron overloading arise from non-transfusion dependent pathologies with increased iron deposition, i.e. thalassemia intermedia, sickle cell anemia, sideroblastic anemia, haemolytic anemia, as well as in alcoholic cirrhosis, porphyria cutanea tarda, and hydiophatic hemochromatosis, the latter being a disturbance of the iron metabolism associated with a mutation of the HLA locus leading to excessive accumulations of iron throughout the body, particularly in parenchymal cells.

The unbalanced iron condition of the above mentioned subjects leads to the progressive iron storage in different body compartments, a well-known clinical condition in transfusion-dependent subjects, which are systematically treated with iron chelating agents. On the other hand, the administration of pharmaceutical compounds capable of depleting iron is far from being routinary on non transfusion-dependent iron overloading subjects.

A specific iron chelation therapy is presently provided by desferoxamine (Desferal®, Novartis). This drug removes iron from various sites, nonetheless, transfusion-dependent subjects are still characterized by rather high residual iron contents, being ferritin (as typical marker) usually within the range of 500-2.000 mcg/ml in plasma, i.e. above the physiologic levels.

Moreover, only in the cirrhotic stage of primary forms of non-transfusive hemochromatosis, an increase in reticuloendothelial iron and serum ferritin is observed, since both iron markers may remain normal in the precirrhotic stage. Furthermore, the only up to now approved available drug for the iron chelation, desferoxamine, needs for a subcutaneous slow infusion to be administered to the patient during 8 to 12 hours, with a marked worsening of life quality. As a matter of fact, subjects with non transfusion-dependent iron overloading are seldom treated for this condition, which in turns causes a variety of degenerative events.

Surprisingly, neither nutritional nor pharmaceutical formulations are available for the treatment of iron overloading. Sometimes vitamin C, vitamin E and selenium, or multivitamin oral formulations are recommended. Nonetheless, such preparations are unspecific, poorly effective and provide unbalanced dosages concerning hemochromatosis.

### Brief summary of the invention

It has now been found that plant polyphenols are useful for the treatment of iron overloading conditions.

The present invention concerns therefore the use of plant polyphenols, substances of catechic and flavonoid structure, for the preparation of medicaments for the treatment of iron overloading conditions. Plant polyphenols can in fact be advantageously administered to iron overloaded subjects, alone or optionally in multivitamin and multimineral oral compositions, to increase their efficacy.

The present invention also concerns compositions comprising catechic- and flavonoid-structure plant polyphenols, optionally in combination with other ingredients, useful for treating the iron overloading conditions occurring in transfusion-dependent and non-transfusion-dependent iron overloaded subjects.

### Detailed disclosure of the invention

Plant polyphenols according to the present invention exist either in the free form (known as aglycone) or as O-derivatives, such as esters (e.g. gallate, acetate, p-coumarate, malonate, etc.) and ethers (e.g. O-methoxy, glycosides), the former being preferred since they apparently display a higher protecting activity towards iron disorders.

The antioxidant effects of polyphenols have been shown in many experimental systems both in vitro (human low-density lipoproteins, liposomes, macrophages, cultured cells) and in tests on healthy human subjects. Several of these compounds promote nitric oxide production by vascular endothelium, inhibit the synthesis of thromboxane in platelets and of leukotriene in neutrophils, modulate the synthesis and secretion of lipoproteins in living animals and human cell lines, and arrest tumour growth as well as inhibit carcinogenesis in different experimental models. The basic mechanism accounting for these effects includes inhibition of phospholipase A₂ and cyclo-oxygenase, inhibition of phosphodiesterase with increase in cyclic nucleotide concentrations, and inhibition of several protein kinases involved in cell signaling.

Preferred plant polyphenols according to the present invention include flavanols, anthocyanins, chalcones, flavandiols, flavanonols, flavanones, flavonols, flavones, isoflavones, hydroxytyrosol.

Flavanols are plant polyphenols widely occurring in vegetal tissues. (+)-Catechin and its stereoisomer (-)-epicatechin, both as aglycones and in the glycosylated and esterified forms, such as gallate and the like, are known with several names (e.g. catechic acid, catechuic acid, cyanidol), being found primarily in higher woody plants, as well as in catechu (gambir and acacia). Proanthocyanidins are a group of compounds comprising, as constitutional units, condensed type catechins. The name "pro-anthocyanidins" is due to their transformation into anthocyanins in aqueous acid environment. The compounds include high molecular procyanidins, prodelphinidin and propelargonidin, and their stereoisomers, dimers, trimers, tetramers, up to decamers of catechins, i.e. their constitutional units. Flavan-3-ols in the afore mentioned forms can be extracted from grape seed, pine bark, green tea, cocoa and many other vegetals rich in catechins in the free, glucosylated, esterified, condensed-polymerized (oligomeric proanthocyanidins, OPC) forms.

The concentration-dependent oxygen free radical scavenging abilities of a grape seed proanthocyanidin extract, compared to vitamin C, vitamin E succinate, superoxide dismutase, catalase and mannitol was assessed by Bagchi D., Garg A., (Res. Commun. Mol. Pathol. Pharmacol., 95(2):179-89, 1997). The activity measured against biochemically generated superoxide anion and hydroxyl radical using a chemiluminescence assay and cytochrome C reduction showed a concentration-dependent inhibition. At 100 mg/l proanthocyanidin inhibits 78-81% of the superoxide anion and hydroxyl radical. Under similar conditions, vitamin C inhibited these two oxygen free radicals by approximately 12-19%, while vitamin E succinate inhibited the two radicals by 36-44%. It follows that proanthocyanidins are more potent scavengers of oxygen free radicals than vitamin C and vitamin E.

Anthocyanins are found in the petals of flowers, in the leaves of many plants and in colored fruits and vegetables. Fruit anthocyanins may be solely in the skins (egg plant, apple), in the whole fruit (cherry, blackberry), or in varying amounts in the skin and flesh of Vitis vinifera (grapes, marc). The chemistry of these materials is based on 2-phenylbenzopyrylium (flavilium) substituted with hydroxy or methoxy groups, namely pelargonidin, cyanidin, delphinidin, petunidin, peonidin, malvidin, which may also be mono, di and tri-saccharides, and position 3 may be acylated, e.g. with p-coumaric acid. Anthocyanins are endowed with capillary protective activity, and the chelating and antioxidant properties of these substances are usefully appliable in the composition of the present invention for the treatment of iron overloading conditions.

Grape derivatives, e.g. marc and wine, also contain an important plant polyphenol, resveratrol, a chalcone flavonoid having strong protecting and anti-inflammatory activities. Resveratrol is the putative building block of oligomeric stilbenes, viniferins, which are even higher inhibitors of the cyclooxygenase activity of prostaglandin H₂ synthase, as well as antifungal agents. In common grapes (Vitis vinifera) the polymeric aggregation gives rise, in its turn, to both viniferins and proanthocyanidins. In other plants, namely Caragana spp (Leguminosae) and Vitis coignetiae (Vitaceae), viniferins are selectively produced, and may therefore be considered as an alternative source of this material for the purposes of the invention.

Isoflavones belong to a class of flavonoids having a wide range of biological, pharmacological and chemoprotective properties against oxygen free radicals. A common dietary source is soybean meal, from where they can be extracted and isolated, as well as other leguminous species containing genistein, genistin, daidzein, daidzin, glycitein, glycitin, and their corresponding 6"-O-malonyl and 6"-O-acetyl esters. Another source of isoflavonoides is liquorice, although liquirrhitigenin and related polyphenols are low hydroxylated isoflavones, therefore considered less active than the soy isoflavones. As a matter of fact, the efficacy of soybean meal in lowering the serum total cholesterol appears to be strictly correlated with its content of isoflavones, which play a role in the prevention of certain cancers by inhibiting protein tyrosine kinase and angiogenesis. Isoflavones induce differentiation in several malignant cell lines, important risk factors in cancer development, as well as inhibiting the growth of androgen-dependent and independent prostatic cancer cells in vitro. Iron overloading is an oxidative stress condition which has proved to stimulate spontaneous tumorigenesis, so that the presence of soybean isoflavones provides both iron scavenging activity and oncological protection in iron overloading.

Hydroxytyrosol (chemically 2-(3,4-dihydroxyphenyl-ethanol) and its derivatives (oleuropein and verbascoside) are bioactive polyphenolis present in olive oil, endowed with both antioxidant and anti-inflammatory properties.

Recent investigations on the effects of the above mentioned components on nitrogen oxide (NO) released in cell cultures and on the activity toward nitric oxide synthase (iNOS) expression, have shown a dose-dependent increase in nitrite production in LPS-challenged mouse macrophages.

Grapes and derivatives thereof (e.g. marc and wine) contain further plant polyphenols, such as flavandiols (dihydroquercetin), flavanonols (myricetin), and flavones (luteolin).

Flavanones and flavonols are known as citrus bioflavonoids because of the high content thereof in *Citrus* spp, although are widely found throughout the plant kingdom. In in vitro experimental models, i.e. the antilipoperoxidant activity in Fe²⁺-induced linoleate peroxidation with detection of conjugated dienes, they thus ranked: rutin > hesperetin > quercetin > naringenin. While flavanones (e.g. hesperetin, naringenin and glycosides) are typically found in citrus fruits, flavonols structures are almost ubiquitary in the plant kingdom. The representative aglycone of flavonols is quercetin, mainly existing as 3-rhamnoside (quercetrin) or 3-rutinoside (rutin), the former being conveniently extracted from Aesculum hippocastanum, the latter occurring in a variety of plants, including tobacco, tea, eucalyptus, grapes, etc.

Plant polyphenols can be found in a variety of other vegetal sources, from which they may be extracted and purified according to known methods. Although a number of plant extracts can provide industrial quantities of the active ingredients, the preferred sources of plant polyphenols, grapes *(Vitis vinifera), Citrus,* soybean and olive *(Olea europaea),* and related by-products can be considered preferred sources of plant polyphenols for the present invention.

The structure-activity relationships of plant polyphenols have also been studied in a variety of in vitro models. Notably, the study of Jorgensen LV; Skibsted LH ("Flavonoid deactivation of ferrylmyoglobin in relation to ease of oxidation as determined by cyclic voltammetry". Free Radic Res, 58(1):335-51 1998) discloses a qualitative relationship between redox potential and rate constants. The apigenin flavone was found more efficient as reducing agent, whereas the absence of a 2,3 double bond in flavanones, the removal of the 4-carbonyl group or the addition of a 3-hydroxy group only has a comparatively minor effect on reactivity. The flavonols are the most efficient reducing agents, effectively reducing MbFe(IV)=O to MbFe(III) and establishing a steady state distribution between the flavonol and MbFe(III) and oxymyoglobin (MbFe(II)O2). Oxidised flavonols reduce MbFe(III) to MbFe(II)O2 very efficiently and much faster than the parent flavonols.

It has now been found that 3',4'-OH substitution on the flavone B-ring plays a crucial role in iron scavenging activity. A still higher OH-substitution is related to an increase in iron chelating ability.

Representative plant polyphenols belonging to the above cited families include:
- flavanols:: catechin, epicatechin, proanthocyanidins;
- anthocyanins:: pelargonidin, cyanidin, delphinidin, petunidin, peonidin, malvidin;
- chalcones:: resveratrol, vitisins, viniferins;
- flavandiols:: dihydroquercetin, dihydrokaempferol [leucocyanidines]; flavanonols: myricetin, (myricitrin), armadendrin, morin;
- flavanones:: hesperetin, (hesperidin), naringenin, (naringin), (narirutin), (neohesperidin);
- flavonols:: quercetin, (quercitrin), (rutin), kaempferol;
- flavones:: apigenin, (7-O-glucoside), rhoifolin, luteolin, (7-O-glucoside);
- isoflavones:: genistein, (genistin), daidzein, (daidzin), glycitein, (glycitin);
- hydroxytyrosol:: hydroxytyrosol, (oleuropein), (verbascoside).

For the considered therapeutic uses, said plant polyphenols may be formulated into suitable administration forms, to be preferably administered by the oral route.

The plant polyphenols may be optionally used in combination with other nutritional supplements, selected from:
- vitamins A, C, E and related substances, i.e. trans/cis alpha- beta- gamma- zeta- carotenes, trans/cis lycopenes, natural retinoids, ascorbic acid and its salts and esters, and the twelve natural occurring isomers of tocopherol and its esters.
- thiolic antioxidants, i.e. alpha-lipoic acid, L-cysteine and its esters, and reduced or oxidized glutathione.
- egg or plant phospholipids, i.e. phosphatidyl choline, phosphatidyl ethanolamine, phosphatidyl glycerol, phosphatidic acid, phosphatidyl inositol, ceramides, and sphyngolipids.
- maltol and ethylmaltol.
- oligoelements, i.e. Zn, Se, Mn, Cr, Mg, V, and other essential non-iron elements, as salts, oxides, chelated or complexed forms.
- pro-antioxidant substances, e.g. vitamins B1, B2, PP, B6, B12, K1, folic acid, pantothenic acid, biotine, and the like, amino acids, alpha hydroxy acids, polyunsaturated fatty acids and conjugated linoleic acid, and ubiquinones (coenzymes Q₆-Q₁₀).

The combination of plant polyphenols with magnesium ions and/or maltol or ethylmaltol is particularly preferred, optionally in admixture with any one of the above mentioned nutritional supplements.

The invention also refers to pharmaceutical and nutritional compositions comprising said combinations as well as suitable carriers. The plant polyphenols may be in form of aglycones, O-glycosides or naturally occurring ethers and esters thereof.

The compositions of the invention preferably comprise at least 200 mg of plant polyphenol per unit dose and at least 250 mg of magnesium ions per unit dose.

The compositions of the invention may be administered by the oral route in the form of sachets, soft or hard gelatin capsules, fast or slow-release tablets or in liquid form.

The compositions can be used as dietary supplements or as pharmaceutical preparations, being preferably orally administered in dosed forms, such as tablets or capsules.

The compositions of the present invention may further include nontoxic inert carriers or diluents in admixture with the above mentioned ingredients. Examples of such non-toxic, inert carriers include wheat starch, silica gel, calcium carbonate, and sodium carboxymethyl cellulose.

The compositions are preferably administered orally in a dose of 1 to 20 mg of plant polyphenols per kg of body weight, the optimum dose of course depending on the body weight of the subject as well as the severity of the iron overloading. Such a daily dosage of the compositions will give the subject the amount of iron sequestration and the inhibition of secondary oxidation, necessary to prevent iron damages.

The daily intake of 1 to 2 tablets or capsules, each containing 250 mg to 1000 mg of active plant polyphenols, is recommended for the treatment of iron overloading in transfused patients, e.g. in thalassemia major, aplastic anemia, congenital anemia, acquired red cell aplasia, myelodisplasia, and in optionally transfusion-dependent thalassemia intermedia and sickle cell anemia and myelodisplasia.

When iron overloading is non transfusion dependent, such as in hemochromatosis or thalassemia intermedia, sickle cell anemia, sideroblastic anemia, haemolitic anemia, alcoholic cirrhosis, porphyria cutanea tarda, and hidiophatic hemochromatosis, the compositions are preferably administered 1-3 times a day in the form of tablets or capsules, each containing from 50 to 400 mg of plant polyphenols, preferably avoiding a total intake exceeding 500 mg per day.

There are no restrictions concerning age and duration of treatment.

The compositions may be in the form of free flowing bulk package or pre-dosed package, such as sachets to be admixed to food or drink, as well as dosed liquid supplements which contain all of the active ingredients of the dietary supplement in unit dosage form.

The following examples further illustrate the invention.

### Example I - Screening of the scavenger activity on in vitro model of iron overloading

The inhibition of hydrogen peroxide by various plant polyphenols is evaluated in an in vitro model.

Chemiluminescence produced by hydroxyl radicals initiated by the ferrous-ascorbate Fenton reaction is measured using a liquid scintillation counter (LKB 1217/1217 Rack Beta), set to the tritium channel. Luminol (5-amino-2,3-dihydro-1,4-phthalazinedione) is dissolved in dimethylsulfoxide and diluted with Krebs-Ringer bicarbonate buffer (pH 7.4) containing 16 µM Hepes. The final concentration of dimethylsulfoxide is 0.06 microlitres/ml.

Under the assay conditions, 3 µM luminol and 100 µM sodium ascorbate-FeSO₄(NH₄)₂ SO₄.6H₂O are mixed with 5 ml of Krebs-Ringer bicarbonate buffer. Chemiluminescence is measured for a 30 second period beginning at 20 seconds after the addition of a quantity of plant polyphenols corresponding to a final concentration of 2 mM.

Polyphenols clearly inhibit the production of free radicals, as herein reported in table 1.

**TABLE 1**

| Inhibition of Fenton-induced peroxides by various plant polyphenols | | | |
|---|---|---|---|
| Plant Polyphenol see (*) | Final concentration of the polyphenol see (*) | Chemiluminescence (cpm) | Percent inhibition % (**) |
| - | (no polyphenols) | 11845.0 +- 1414.0 | 0 |
| a | 2 µM | 158.9 +- 34.2 | 98.5 |
| b | 2 µM | 616.6 +- 34.1 | 95 |
| c | 2 µM | 2082 +- 401.6 | 82 |
| d | 2 µM | 4829.3 +- 792.1 | 60 |
| e | 2 µM | 3350.1 +- 498.4 | 72 |

| | | | |
|---|---|---|---|
| (*) The source, concentration and origin of the plant polyphenols is listed hereinafter: a) flavanols (catechins and proanthocyanines) source: grape seed; content: 90% purity; origin: Vitavin® by Laboratorio Centroflora Ltd (Sao Paulo, Brazil) b) flavanones and flavonols source: lemon peel, content: 30% purity; origin: Lim30Bio® by Pharmachem Labs, Inc. (USA) c) anthocyanin, chalcons, flavandiols, flavanonols and flavones source: marc, content: 60% purity; origin: Aldeina/R® by Enocianina Fornaciari srl (RE, Italia) d) isoflavones Source: flour Soya, content: 70% purity; origin: Slogan 70® by SPE, Inc. (USA) e) Hydroxytyrosol source: waste water from olive oil, content: 85% purity; origin: Institute of Pharmacological Science, University of Milan (I) | | | |
| (**) % inhibition is expressed as percent of free radical production inhibited by plant polyphenols as measured by changes in chemiluminescence in comparison with control. | | | |

### Example II - Oral composition with citrus polyphenols

A composition is prepared in the form of capsules, with the following ingredients:

| | |
|---|---|
| flavanones and flavonols, (b) in Example I | 500 mg |
| calcium carbonate | 250 mg |
| magnesium hydroxide | 160 mg |
| zinc subcarbonate | 15 mg |
| beta-carotene | 5 mg |
| alpha-tocopherol | 6 mg |

with the balance a nutritionally acceptable carrier.

### Example III - Oral composition with soybean isoflavones and olive polyphenols

A composition is prepared with the ingredients of Example II except that 500 mg of flavanones and flavonols (b) are replaced by 250 mg of soybean isoflavones (d) and 250 mg of hydroxytyrosol (e) as indicated in Example I.

### Example IV - Oral composition with grape seed polyphenols

A composition is prepared in the form of capsules with the following ingredients:

| | |
|---|---|
| flavanols, (a) in Example I | 300 mg |
| magnesium hydroxide | 160 mg |
| zinc subcarbonate | 25 mg |
| selenium yeast (2000 mcg/g) | 5 mg |
| conjugated linoleic acid | 30 mg |
| licopene 10% | 20 mg |
| alpha-tocopherol | 12 mg |

with the balance a nutritionally acceptable carrier.

### Example V - Oral composition with grape skin anthocyanins

A composition is prepared in the form of ampoules with the following ingredients:

| | |
|---|---|
| anthocyanin, chalcons, flavandiols, flavanols and flavones, (c) in Example I | 400 mg |
| ascorbic acid | 12 mg |
| maltol | 30 mg |
| L-tartaric acid | 15 mg |
| strawberry flavor | 2 mg |

solubilizing the mixture in distilled water qs to 250 ml.

### Example VI - Polyvitamin and mineral oral composition with a pool of plant polyphenols

The following composition is prepared in the form of 1250 mg capsules:

| INGREDIENTS | CONTENT |
|---|---|
| (a) flavan-3-ols and its oligomers (proanthocyanidins, OPC) | 50 mg |
| (b) flavanones and flavon-3-ols | 20 mg |
| (c) anthocyanines, chalcons and its oligomers (viniferins) | 150 mg |
| (d) isoflavons | 5 mg |
| alpha-lipoic acid | 10 mg |
| reduced glutathione | 5 mg |
| vitamin C (calcium ascorbate) | 45 mg |
| vitamin E (alpha-tocopherol) | 30 mg |
| maltol | 50 mg |
| folic acid | 0.05 mg |
| vitamin A | 0.767 mg |
| beta-carotene | 0.2 mg |
| vitamin B₁ | 0.35 mg |
| vitamin B₂ | 0.4 mg |
| vitamin B₆ | 0.5 mg |
| vitamin B₁₂ | 0.75 mcg |
| vitamin PP (nicotinamide) | 4.5 mg |
| vitamin K₁ | 1.75 mcg |
| coenzyme Q₁₀ | 2.5 mg |
| panthothenic acid (calcium panthothenate) | 1.5 mg |
| vitamin H (biothine) | 37.5 mg |
| magnesium (Mg hydroxyde) | 180 mg |
| manganese (Mn gluconate) | 5 mg |
| zinc (Zn subcarbonate) | 15 mg |
| selenium (Se yeast) | 27.5 mg |

with the balance a nutritionally acceptable carrier, with cocoa butter and phospholipids incorporating thiolic substances (glutathione and lipoic acid) in order to decrease any unpleasant off-flavors.

NB Ingredients (a), (b), (c), (d) and (e) as listed in Example I

### Example VII - Clinical trials on beta-thalassemia patients - restoration of lipid antioxidants

A group of 7 beta-thalassemia patients, aged 18 to 29 years were monitored for their level of lipid-soluble antioxidants according to the procedures of Maggio A., et al., Blood, 88(9):3608-14 (1996).

The trial consisted in one month administration of 1 capsule per day of the nutritional supplement of Example V. Before starting the treatment, serum levels of vitamin E are inversely correlated with ferritin (r = -0.45; P = 0.003), suggesting a major consumption of this antioxidant under iron overloading. Nontransferrin bound iron (NTBI) in the range 4.5 to 54.8 mcg/dl (mean, 21.8 +/- 13.9) had a positive trend with ferritin (r = 0.37, P = 0.03).

As control patients, two non-thalassemia subjects with HCV-related chronic hepatitis have been monitored.

Serum levels of lipid-soluble antioxidants, namely vitamin E, vitamin A and lycopene (r = -0.47, P = 0.020), inversely correlated with the levels of transaminases, are partially restored after the once-a-day administration of the oral composition of the Example VI, as reported in table 2, for 30 days.

**TABLE 2**

| Partial restoration of antioxidant levels in beta-thalassemia subjects | | | | |
|---|---|---|---|---|
| Subjects | vitamin E (µM/l) | vitamin A (µM/l) | beta-carotene (µM/l) | lycopene (µM/l) |
| beta-thalassemia | 1.05+-0.25 | 10.7+-3.1 | 0.29+-0.05 | 0.18+-0.04 |
| level at day 0 (t₀) | | | | |
| beta-thalassemia | 1.59+-0.58 | 18.6+-4.3 | 0.54+-0.16 | 0.49+-0.21 |
| level at day 30 (t₃₀) | | | | |
| control | 1.71+-0.4 | 19.0+-0.5 | 0.41+-0.06 | 0.56+-0.18 |
| difference % at t₀ (*) | -39% | -44% | -44% | -68% |
| difference % at t₃₀ (**) | -7% | -2% | +10% | -13% |

| | | | | |
|---|---|---|---|---|
| (*) t₀ = before treatment. | | | | |
| (**) t₃₀ = after 30 days treatment with oral composition of Example V. | | | | |

Total serum antioxidant potential, measured as trolox equivalent antioxidant capacity significantly increases from -14%.to -5% after the one-month treatment with the composition of Example VI. The content of lipid soluble vitamins is restored to the pseudo-physiologic parameters, taking into account the previous hepatic condition.

### Example VIII - Clinical trial on beta-thalassemia maior - decrease in oxygenated radicals in blood.

The oxidative condition in 8 transfusion-dependent, beta-thalassemia major patients was monitored before and during administration of one capsule pro die of the composition according to Example V, by means of the d-ROMs kit test® (Gallarati-IRAM, Parma, Italy).

Briefly, 40 microlitres of blood sample are taken by finger puncture at day 0, 5, 10, and 20 of the oral treatment. The analysis is carried out immediately after the sampling, the 40 microlitres capillary is placed in 3,92 ml of acetate buffer solution at pH 4.8 containing 40 microlitres of N,N-diethyl-para-phenylenediamine. After dissolving in the aqueous media and adding one drop of the enzyme solution, the sample is centrifuged at 3000 rpm for 3 minutes, then placed in a cuvette and heated at constant temperature for 3 minutes, then the absorbance is measured at 505 nm. The results are shown in table 3.

**TABLE 3**

| Decrease in oxygenated radicals in blood on beta-thalassemia maior subjects | | | | |
|---|---|---|---|---|
| | Mean value at day 0 (*) | Mean value at day 5 (*) | Mean value at day 10 (*) | Mean value at day 20 (*) |
| Control | 301 | 281 | 248 | 239 |
| Patients | 593 | 430 | 327 | 285 |

| | | | | |
|---|---|---|---|---|
| (*) The oxidative stress is expressed as Carratelli Units (Carr.U.), whereas 1 Carr.U. equals approximately the concentration of 0.08 mg % of peroxide. | | | | |

The trial shows that the levels of oxidative stress measured are partially restored to the standard values.

### Example IX - Clinical trial on hydiopatic hemochromatosis - decrease in blood oxygenated radicals

The same test of Example VIII is carried out on 6 patients with detected hidiopatic hemochromatosis. The results are shown in table 4.

**TABLE 4**

| Decrease in oxygenated radicals in blood subjects on hidiopatic hemochromatosis subjects | | | | |
|---|---|---|---|---|
| | Mean value at day 0 (*) | Mean value at day 5 (*) | Mean value at day 10 (*) | Mean value at day 20 (*) |
| Control (**) | 301 | 281 | 248 | 239 |
| Patients | 486 | 402 | 335 | 279 |

| | | | | |
|---|---|---|---|---|
| (*) The oxidative stress is expressed as Carratelli Units (Carr.U.), whereas 1 Carr.U. equals approximately the concentration of 0.08 mg % of hydrogen peroxide. | | | | |
| (**) Same control data as per Example VIII are reported. | | | | |

## Claims

1. The use of plant polyphenols for the manufacture of a medicament for the treatment of iron overloading.

2. The use according to claim 1 wherein said plant polyphenols are selected from flavanols, anthocyanins, chalcones, flavandiols, flavanonols, flavanones, flavonols, flavones, isoflavones, hydroxytyrosol.

3. The use according to claim 2 wherein said plant polyphenols are selected from the group consisting of catechin, epicatechin, proanthocyanosides, pelargonidin, cyanidin, delphinidin, resveratrol, dihydroquercetin, dihydrokaempferol, myricetin, armadendrin, morin, hesperetin, naringenin, quercetin, kaempferol, apigenin, luteolin, genistein, daidzein, glycitein, hydroxytyrosol or mixtures thereof.

4. The use according to claim 3 wherein said plant polyphenols are O-glycosides, or naturally occurring esters and ethers thereof.

5. The use according to claims 1-4, wherein said iron overloading derives from blood transfusion-dependent therapy in thalassemia major, aplastic anemia, congenital anemia, acquired red cell aplasia, myelodisplasia, transfusion-dependent thalassemia intermedia and sickle cell anemia.

6. The use according to claim 1 wherein said iron overloading derives from non transfusion-dependent thalassemia intermedia, sickle cell anemia, sideroblastic anemia, haemolytic anemia, alcoholic cirrhosis, porphyria cutanea tarda, and hidiophatic hemochromatosis.

7. Pharmaceutical or nutritional compositions comprising as the active ingredient a plant polyphenol in combination with magnesium ions and/or with maltol or ethylmaltol and suitable carriers.

8. Pharmaceutical or nutritional compositions according to claim 7 comprising at least 200 mg of plant polyphenols per unit dose.

9. Pharmaceutical or nutritional compositions according to claim 8 comprising at least 250 mg of magnesium ion.

10. Pharmaceutical or nutritional compositions according to any one of claims 7-9 wherein the plant polyphenols are selected from the group consisting of catechin, epicatechin, proanthocyanosides, pelargonidin, cyanidin, delphinidin, resveratrol, dihydroquercetin, dihydrokaempferol, myricetin, armadendrin, morin, hesperetin, naringenin, quercetin, kaempferol, apigenin, luteolin, genistein, daidzein, glycitein, hydroxytyrosol or mixtures thereof.

11. Pharmaceutical or nutritional compositions according to claim 10, wherein said plant polyphenols are O-glycosides or naturally occurring esters and ethers thereof.

12. Pharmaceutical or nutritional compositions according to any one of the previous claims, further comprising nutritionally acceptable ingredients selected from the group consisting of ascorbates, carotenoids, tocopherols, ubiquinones, phospholipids, alpha-lipoic acid, glutathione, minerals, vitamins and pro-vitamins, amino acids, alpha hydroxy acids, polyunsaturated fatty acids and conjugated linoleic acid, or mixtures thereof.

13. Pharmaceutical or nutritional compositions according to any one of claims 7-12 for the oral administration in the solid form such as sachets, soft or hard gelatin capsules, fast or slow release tablets, or in the liquid form.
